## Europäisches Patentamt

## European Patent Office

## Office européen des brevets

(19)

(11) Veröffentlichungsnummer: **0 011 334**
**B1**

(12)

# EUROPÄISCHE PATENTSCHRIFT

(45) Veröffentlichungstag der Patentschrift:
09.02.83

(51) Int. Cl.³: **C 12 P 7/06**

(21) Anmeldenummer: 79200648.8

(22) Anmeldetag: 06.11.79

(54) Verfahren zur kontinuierlichen Herstellung von Alkohol durch Fermentation.

(30) Priorität: 13.11.78 CH 11637/78

(43) Veröffentlichungstag der Anmeldung:
28.05.80 Patentblatt 80/11

(45) Bekanntmachung des Hinweises auf die Patenterteilung:
09.02.83 Patentblatt 83/6

(84) Benannte Vertragsstaaten:
AT CH DE FR IT SE

(56) Entgegenhaltungen:
FR-A-927 156
GB-A-174 955
US-A-2 440 925
US-A-3 109 782

(73) Patentinhaber: Process Engineering Company SA, Alte Landstrasse 415, CH-8708 Männedorf (CH)

(72) Erfinder: Müller, Hans, Dr. Ing., Im Allmendli, CH-8703 Erlenbach (CH)
Erfinder: Müller, Felix, Dr., Bahnhofstrasse 46, CH-8712 Stäfa (CH)

(74) Vertreter: Herrmann, Peter, Chemap AG Alte Landstrasse 415, CH-8708 Männedorf (CH)

## Verfahren zur kontinuierlichen Herstellung von Alkohol durch Fermentation

Die Erfindung betrifft ein Verfahren zur kontinuierlichen Herstellung von Alkohol, insbesondere von Ethanol durch Gärung in einem Fermenter.

Verfahren zur Herstellung von Alkohol durch Fermentation sind im allgemeinen bekannt. Es ist nach G. R. Cysewski und Ch. R. Wilke, Biotechn. Bioeng. 19, 1125—1143 (1977) auch bekannt, Ethanol während der Gärung aus dem Fermenter mittels Vakuum abzuziehen, wodurch eine Erhöhung der Ausbeute an Alkohol durch die Aufhebung der Alkoholinhibierung, die bei höherer Konzentration auftritt, erzielt wird. Um die gewünschte Produktivitätserhöhung zu erreichen, wird bei einem Gesamtdruck von 50 mm Hg (66,7 mbar) gearbeitet.

In einem kontinuierlichen Verfahren gemäß US-A-2 440 925 zur Herstellung von Alkohol wird ein Teil der gärenden Maische zusammen mit der darin enthaltenen Hefe außerhalb des Fermenters in einer Strippingkolonne im Vakuum von Alkohol befreit und in den Fermenter zusammen mit der Hefe zurückgeführt. Es wird unter einem Vakuum von 34 mm Hg (45,3 mbar) gearbeitet.

Solch niedrige Drücke haben jedoch den Nachteil, daß große Fermenter vakuumfest hergestellt werden müssen. Hierdurch werden an Dichtsysteme und an den abnahmepflichtigen Behälter unverhältnismäßig hohe Anforderungen gestellt. Durch Anlegen von Vakuum an den Fermenter wird auch die während der kontinuierlichen Gärung erforderliche Sterilität nicht mehr gewährleistet, da an undichten Stellen durch Unterdruck fremde Mikroorganismen in den Behälter gelangen können. Das gleiche gilt auch beim Anlegen von Vakuum an die Strippingkolonne.

Neben der diskontinuierlichen Arbeitsweise zeigt das Verfahren nach US-A-2 440 925 weitere Nachteile. Obwohl die Strippingkolonne unter Vakuum und bei nur 36,7° C betrieben wird, erfolgt die Inhibierung der Hefe durch die darin auftretenden hohen Alkoholkonzentrationen in der Dampfphase; Alkoholdämpfe töten die Hefezellen ab. Das bekannte Verfahren vermag daher die Aufgabe der Vermeidung des toxischen Effektes zu hoher Alkoholkonzentrationen auf die Gärungsorganismen nicht zu lösen. Auch die starke Verschmutzung des Verdampfers und der Strippingkolonne durch die Hefe ist als Nachteil anzusehen.

Aufgabe der Erfindung ist es, ein kontinuierliches Verfahren zur Herstellung von Ethanol durch Gärung zu schaffen, das die aufgezeigten Nachteile umgeht und dennoch eine Inhibierung der Gärung durch ansteigende Alkoholkonzentrationen vermeidet und die Vitalerhaltung der Gärungsorganismen gewährleistet.

Erfindungsgemäß wird diese Aufgabe durch ein Verfahren zur kontinuierlichen Herstellung von Ethanol durch Gärung nach Patentanspruch 1 gelöst, das dadurch gekennzeichnet ist, daß die Hefezellen aus der vergorenen Maische mittels einer Zentrifuge unmittelbar vor dem Eintritt in einen Verdampfer abgetrennt und teilweise in den Fermenter rezirkuliert werden.

Der Fermenter selbst kann unter Normaldruck oder leichtem Unter- oder Überdruck betrieben werden. Unter leichtem Unterdruck sind hierbei Drücke nicht unter 0,5 bar und unter Überdruck solche nicht über 0,5 bar zu verstehen. Das Nährsubstrat wird dem Fermenter kontinuierlich zugeführt, und die Fermentierung erfolgt bei konstanter Temperatur. Dem Fermenter wird über einen Kreislauf stetig ein Teilstrom der Maische an einer Stelle entnommen und nach vollständigem oder teilweisem Entzug des Alkohols teilweise wieder zugeführt. Die Entnahme für den Kreislauf ist in der Regel ein Vielfaches der Substratmenge, die zugeführt wird. Die effektive Entnahme aus dem Fermenter, d. h. die Menge der Ausschleusung entspricht der Menge an frisch zugeführtem Substrat, so daß der Inhalt im Fermenter stets konstant bleibt.

Der Entzug des Alkohols aus der Maische erfolgt außerhalb des Fermenters in einem Vakuumverdampfer bei Temperaturen von 18—45° C, vorzugsweise bei Gärtemperatur. Das Vakuum wird mittels eines Thermokompressors erzeugt, welcher die Brüden verdichtet und kondensiert und die dabei freigewordene Wärmemenge dem Verdampfer wieder zur Verfügung stellt. Gleichzeitig erzeugt der Thermokompressor das erforderliche Vakuum.

Die Hauptmenge der aus der Gärung kommenden Kohlensäure wird dem Fermenter direkt entnommen. Der Rest der noch in der Maische enthaltenen Kohlensäure wird vor dem Thermokompressor ausgetrieben.

Die Abtrennung der Hefezellen kann unmittelbar nach dem Fermenter, also vor dem Abtrennen des Alkohols erfolgen. Die Abtrennung vor dem Verdampfer hat den Vorteil, daß der Verdampfer weniger rasch verschmutzt. Ein weiterer Vorteil ist darin zu sehen, daß die Hefezellen unmittelbar nach der Abtrennung bei gleicher Temperatur in den Fermenter zurückgeführt werden können und so den Biomasseanteil erhöhen. Gleichzeitig kann bei dieser Arbeitsweise der Verdampfer bei höherer Temperatur als der Gärtemperatur betrieben werden. Dies kann wünschenswert sein, wenn man bei Anwendung der Thermokompression das Kompressionsverhältnis den thermischen Erfordernissen der Verdampfung optimal anpassen will.

Es ist erforderlich, daß ein Teil des Substrates, in welchem sich im Laufe längerer Fermentierperioden Giftstoffe anreichern, abgeschlammt wird.

Das Verfahren soll anhand eines schematischen Fließbildes näher erläutert werden.

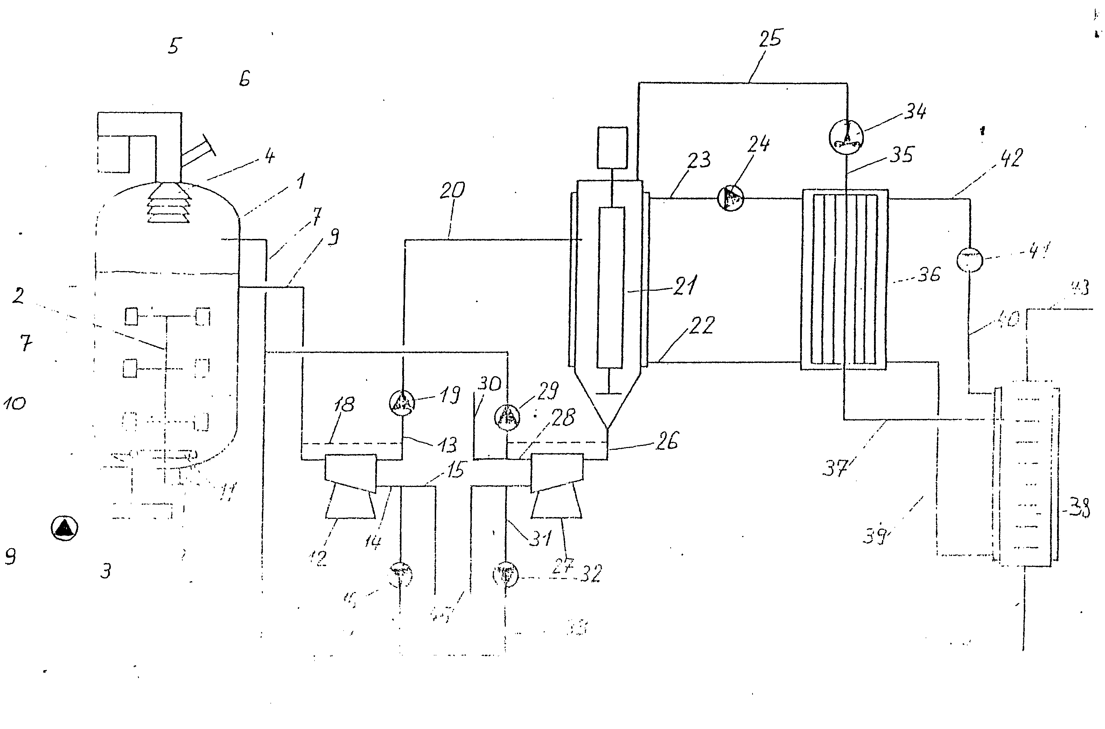

Die einzige Figur zeigt einen Fermenterbehälter 1. Dieser ist mit einem Rührer 2 versehen, der seinerseits durch einen Elektromotor 3 angetrieben wird. Im Dom des Fermenters 1 ist ein mechanischer Schaumabscheider 4 angebracht, der ebenfalls von einem Elektromotor 5 angetrieben wird. Der Schaumabscheider 4 besitzt einen Stutzen 6. Im oberen Teil des Fermenters 1 ist über einen Stutzen eine Leitung 7 angebracht, an deren Ende eine Pumpe 8 installiert ist. Auf etwa gleicher Höhe wie Leitung 7, jedoch auf entgegengesetzter Seite, ist ein Stutzen mit einer Leitung 9 verbunden. Ein weiterer Stutzen verbindet eine Luftleitung 10 mit dem Fermenter 1, die in einem Luftverteiler 11 innerhalb des Fermenterkessels mündet. Am Ende der Leitung 9 ist eine Zentrifuge 12 angeschlossen, die eine Abgangsleitung 13 für die flüssige Phase und eine Abgangsleitung 14 für die konzentrierte Phase aufweist. Von der Leitung 14 zweigt die Leitung 15 ab. Zwischen der Leitung 14 und 17 ist eine Pumpe 16 installiert. Zur Umgehung der Zentrifuge ist ein Bypass 18 mit den erforderlichen, nicht gezeigten Ventilen vorgesehen. Eine Pumpe 19 ist zwischen den Leitungsstücken 13 und 20 installiert. Der Verdampfer 21, der vorzugsweise ein Fallfilmverdampfer ist, ist an eine Zufuhrleitung 22 für das Heizmedium und eine Abfuhrleitung 23 angeschlossen, die ihrerseits eine Pumpe 24 beinhaltet. Vom Kopf des Verdampfers führt eine Brüdenleitung 25 und vom Sumpf eine Leitung 26 entweder direkt zurück zum Fermenter 1 oder auf eine Zentrifuge 27. Von der Zentrifuge 27 führt eine Leitung 28 über eine Pumpe 29 auf die Leitung 7. Von der Leitung 28 zweigt eine weitere Leitung 30 in ein nicht gezeigtes Kanalsystem. Die Konzentratleitung 31 führt über eine Pumpe 32 und das Leitungsstück 33 zum Fermenter 1 zurück. An die Leitung 25 ist ein Thermokompressor 34 angeschlossen und über die Leitung 35 mit einem Wärmeaustauscher 36 verbunden. Aus dem Wärmeaustauscher 36 führt eine Produktleitung 37 auf eine Rektifizierkolonne 38. Die Rektifizierkolonne 38 weist eine Zuleitung 39 für das Heizmedium und eine Ableitung 40 auf, die über eine Pumpe 41 und das Rohrleitungsstück 42 in den Wärmetauscher 36 mündet. Eine Leitung 43 ist für den rektifizierten Alkohol und eine Leitung 44 für das verbliebene Heißwasser vorgesehen.

Im Betrieb wird im Fermenterkessel 1 die alkoholische Gärung durchgeführt. Über die Pumpe 8 und die Leitung 7 wird frisches Substrat in den Fermenter zudosiert. Die an sich anaerobe Gärung wird zeitweise mit sehr geringen Luftmengen von 0,1 – 0,2 vvm (Volumen Luft pro Volumen Fermenterinhalt pro Minute) über die Leitung 10 und den Luftverteiler 11 zur Regenerierung der Hefezellen begast. Während der Gärung bewirkt das Rührwerk 2, welches durch den Elektromotor 3 angetrieben wird, eine ständige Umwälzung des Behälterinhaltes, da die Umwälzung durch das entstehende Kohlensäuregas im allgemeinen nicht ausreicht. Die entstehende Kohlensäure wird über den Gasabzugstutzen 6 des mechanischen Schaumabscheiders 4 kontinuierlich abgezogen und kann einer Weiterverwendung zugeführt werden. Aus dem Fermenter 1 wird kontinuierlich ein Teilstrom über die Leitung 9 entnommen und in der Zentrifuge 12 von der Hefe befreit. Die Hefe wird zum größten Teil durch die Leitung 14 über die Pumpe 16, den Rohrleitungsteil 17 und die Sammelleitung 33 in den Fermenter geführt. Ein Teil der Hefe wird, abhängig von der Konzentration im Fermenter 1, über die Leitung 15 ausgeschleust und kann einer Aufbereitung als Futterhefe zugeführt werden. Ein Stutzen 9' dient zur vollständigen Entleerung des Fermenters. Die enthefte Maische wird der Zentrifuge 12 über das Leitungsstück 13 mittels Pumpe 19 und Leitung 20 in den Verdampfer 21 gefördert. Im Verdampfer wird unter Vakuum, je nach Arbeitsdruck, ein Brüden als Azeotrop aus Alkohol und Wasser über die Leitung 25 dem Thermokompressor 34 zugeführt. Im Thermokompressor 34 wird in bekannter Weise der Brüden verdichtet. Die bei der Verdichtung entstehende Wärme wird dem Alkohol/Wasser-Gemisch in einem Wärmeaustauscher 36, der gleichzeitig als Kondensator wirkt, entzogen und einem Wärmeübertragungsmedium, beispielsweise Wasser, zugeführt. Dieses zirkuliert über die Leitung 22, den Verdampfer 21 und nach Abgabe der zur Verdampfung des Azeotropes erforderlichen Wärmemenge über die Leitung 23, die Pumpe 24 zurück zum Wärmeaustauscher 36.

Das Gemisch aus Alkohol und Wasser wird vom Wärmeaustauscher der Rektifikationskolonne 38 über die Leitung 37 zugeführt. Die Beheizung der Kolonne 38 erfolgt mittels Wärmeübertragungsmedium vom Wärmeaustauscher 36 über die Leitung 39, welches über Leitung 40, Pumpe 41, Leitung 42 und Leitung 22 direkt dem Verdampfer 21 zugeführt werden kann. Bei Verwendung der Zentrifuge 27 wird der Sumpf aus dem Verdampfer zum größten Teil über die Leitung 7 in den Fermenter 1 zurückgeführt und nur ein Teil der Kanalisation zugeführt.

**Patentansprüche**

1. Verfahren zur kontinuierlichen Herstellung von Ethanol durch Gärung in mindestens einem Fermenter, wobei während der Gärung aus dem Fermenter ein Teilstrom der vergorenen Maische kontinuierlich abgezogen, außerhalb des Fermenters unter Vakuum Alkohol abgetrennt und Maische in den Fermenter zurückgeführt wird, dadurch gekennzeichnet, daß die Hefezellen aus der vergorenen Maische mittels einer Zentrifuge unmittelbar vor dem Eintritt in einen Verdampfer (21) abgetrennt und teilweise in den Fermenter (1) rezirkuliert werden.

2. Verfahren nach Patentanspruch 1, dadurch gekennzeichnet, daß die Entfernung des Alkohol-Wassergemisches durch Verdampfen (21) bei Temperaturen von 18°C bis 45°C, vorzugsweise bei Gärtemperatur, erfolgt.

## Claims

1. Process for the continuous production of ethanol by fermentation in one or more fermenters, continuously removing during fermentation a patial stream of the fermented mash from the fermenter, removing alcohol form the partial stream in a vacuum by evaporation, external of the fermenter, and returning mash to the fermenter, characterized by the yeast cells being separated from the fermented mash by centrifugation, immediately before the entry into the evaporator (21) and partially returned to the fermenter (1).

2. Process according to claim 1, wherein the step of removing alcohol is carried out by evaporation of the alcohol-water mixture at temperatures from 18 until 45°C, preferably at fermentation temperature.

## Revendications

1. Procédé de production en continu d'éthanol par fermentation dans un ou plusieurs fermenteurs avec soutirage continu pendant la fermentation d'une fraction du moût fermenté à l'extérieur du fermenteur par action du vide, et retour du moût débarrassé de l'éthanol dans le ou les fermenteurs; caractérisé en ce que les cellules de levure sont séparées du moût fermenté (par centrifugation) immédiatement avant l'entrée dans un évaporateur (21) et en partie renvoyées dans le ou les fermenteurs (1).

2. Procédé selon la revendication 1, caractérisé en ce que l'élimination du mélange alcool-eau est réalisé par évaporation (21) à des températures de 18−45°C, particulières à la température de fermentation.